# EUROPEAN PATENT APPLICATION

(11) **EP 3 574 920 A1**
(43) Date of publication of application: **04.12.2019**
(21) Application number: 18305672.0
(22) Date of filing: 31.05.2018
(51) Int. Cl.: A61K 45/06, A61K 31/5415, A61P 35/00

(54) **NUPR1 INHIBITION FOR TREATING CANCER**

(71) Applicant: INSTITUT NATIONAL DE LA SANTE ET DE LA RECHERCHE MEDICALE (INSERM), 75013 Paris (FR); Rizzuti, Bruno, 87036 Rende (IT)
(72) Inventor: RIZZUTI, Bruno, 87036 RENDE (IT); IOVANNA, Juan, 13288 MARSEILLE (FR); NEIRA, Jose Luis, 03202 ELCHE, ALICANTE (ES); XIA, Yi, Shapingba District, Chongqing 401331 (CN); SANTOFIMIA-CASTAÑO, Patricia, 13288 MARSEILLE (FR); ABIAN, Olga, 50018 SARAGOSSE (ES); PENG, Ling, 13009 MARSEILLE (FR); VELAZQUEZ CAMPOY, Adrian, 50018 SARAGOSSE (ES)
(74) Representative: Nony

(57) **Abstract**

The present invention relates to the treatment of a tumor selected from the group consisting of pancreatic cancer, liver cancer, melanoma, colon cancer, glioblastoma, osteosarcoma, prostate cancer and breast cancer, more particularly pancreatic cancer.

The inventors found a novel family compounds of formula (I) able to treat a variety of cancers by inhibiting NUPR1, a stress-inducible 82-amino-acid-long, intrinsically disordered member of the AT-hook family of chromatin proteins. Thus, the present invention relates to a compound of formula (I) for its use in the treatment of a tumor selected from the group consisting of pancreatic cancer, liver cancer, melanoma, colon cancer, glioblastoma, osteosarcoma, prostate cancer and breast cancer. In particular, the inventors tested compounds of formula (I) on four well characterized primary pancreatic cancer-derived cells 02.063 and LIPC (Basal subtype), Foie8b (derived from a liver metastasis) and HN14 (Classical subtype), as well as on other cellular lines derived from different tumors, i.e. U87 (glioblastoma), A375 and B16 (melanoma), U2OS and SaOS (osteosarcoma), HT29, SK-CO-1 and LS174T (colon cancer), HepG2 (hepatocarcinoma), PC3 (prostate) and MDA-MB-231 (breast cancer).

The compounds *per se* are also claimed.

## Description

### FIELD OF THE INVENTION

The invention relates to the NUPR1 inhibitors and to their use as drugs, in particular as anti-cancer drugs.

The invention in particular concerns the use of these compounds in the treatment of a tumor selected from the group consisting of pancreatic cancer, liver cancer, melanoma, colon cancer, glioblastoma, osteosarcoma, prostate cancer and breast cancer, more particularly pancreatic cancer.

### BACKGROUND OF THE INVENTION

Cancer is a major burden of disease worldwide. Each year, tens of millions of people are diagnosed with cancer around the world, and more than half of the patients eventually die from it. Globally, nearly 1 in 6 deaths is due to cancer.

In many countries, cancer ranks the second most common cause of death following cardiovascular diseases. With significant improvement in treatment and prevention of cardiovascular diseases, cancer has or will soon become the number one killer in many parts of the world. As elderly people are most susceptible to cancer and population aging continues in many countries, cancer will remain a major health problem around the globe.

This awareness led the World Health Assembly to pass, in 2017, the resolution *Cancer Prevention and Control through an Integrated Approach* to urge governments and WHO to accelerate action to achieve the targets specified in the *Global Action Plan* and *2030 UN Agenda for Sustainable Development* to reduce mortality from cancer.

In spite of the recent advances, there remains a need in the art for novel anti-cancer drugs either as simple alternatives to already available drugs or having better properties than the drugs already available. These better properties can have a more effective activity against tumors and/or a smaller or absent risk of developing unwanted side effects.

Accordingly, there remains a need for compounds for use in the treatment of cancers.

In particular, there remains a need for novel compounds able to treat a variety of cancers and more particularly cancers selected from the group consisting of pancreatic cancer, liver cancer, melanoma, colon cancer, glioblastoma, osteosarcoma, prostate cancer and breast cancer, more particularly pancreatic cancer.

There also remains a need for anti-cancer compounds having no side effects at an effective dose when used in a patient in need thereof. In particular, there remains a need for anti-cancer compounds having no neurological side effects at an effective dose when used in a patient in need thereof

The invention herein described has for purpose to meet the aforementioned needs.

### SUMMARY OF THE INVENTION

The present invention relates to compounds having inhibitory activity for NUPR1 and to their use in the treatment of cancers.

Accordingly, an object of the present invention is a compound of formula (I): 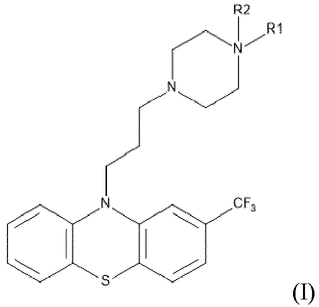 wherein:
R₁ represents a linear or branched (C₂-C₅)alkyl group, optionally substituted with a -NR₃R₄ group;
R₂ represents a non-bonding pair or a -(CH₂)₁₋₅NR₃R₄ group, wherein when R₂ does not represent a non-bonding pair, then the nitrogen atom to which R₂ is attached is positively charged; and
R₃ and R₄ independently represent:
   - a hydrogen atom;
   - a -OH group;
   - a linear or branched (C₁-C₆)alkyl group, said alkyl group being optionally substituted with one or several substituents chosen from a halogen atom, a -OH group and a -SH group;
   - a linear or branched (C₁-C₆)alkoxy group, said alkoxy group being optionally substituted with one or several substituents chosen from a halogen atom, a -OH group and a -SH group; or
   - a -COOH group;
or R₃ and R₄ form, together with the nitrogen atom to which they are attached, a saturated or unsaturated (3- to 6-membered)heterocycle comprising, in addition to the nitrogen atom to which R₃ and R₄ are attached, 0, 1 or 2 additional heteroatom(s) chosen from a nitrogen atom and an oxygen atom; said heterocycle being optionally substituted with one or several substituents chosen from a halogen atom, a -OH group, a (=O) group and a linear or branched (C₁-C₆)alkyl group;
said compound of formula (I) being in all the possible tautomeric and isomeric forms: racemic, enantiomeric and diastereoisomeric, and also as addition salts with inorganic and organic acids or with inorganic and organic bases of the compound of formula (I);
for its use in the treatment of a tumor selected from the group consisting of pancreatic cancer, liver cancer, melanoma, colon cancer, glioblastoma, osteosarcoma, prostate cancer and breast cancer.

In a particular embodiment, R₁ represents:
- a -C₂H₅ group; or
- a -(C₂H₄)NR₃R₄ group, with R₃ and R₄ being as defined above.

In a further embodiment, the compound for use according to the invention is of formula (II): 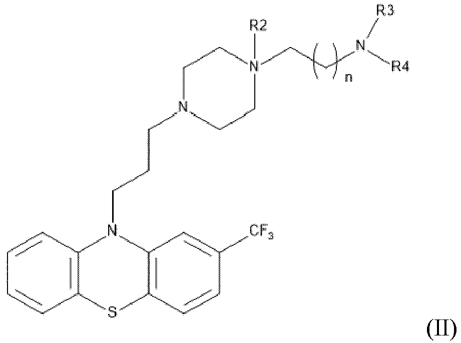 wherein:
n is an integer selected from the group ranging from 0 to 4; and
R₂, R₃ and R₄ are as previously defined.

In an embodiment of the invention, n present in formula (II) represents 1.

In another embodiment, R₃ and R₄ independently represent a linear or branched (C₁-C₆)alkyl group or R₃ and R₄ form, together with the nitrogen atom to which they are attached, a saturated or unsaturated (3- to 6-membered)heterocycle comprising, in addition to the nitrogen atom to which R₃ and R₄ are attached, 0, 1 or 2 additional heteroatom(s) chosen from a nitrogen atom and an oxygen atom; said heterocycle being optionally substituted with one or several substituents chosen from a halogen atom, a -OH group, a (=O) group and a linear or branched (C₁-C₆)alkyl group.

In a particular embodiment, R₃ and R₄ independently represent:
- a -(CH₂) group; or
- a -(C₂H₅) group;
or form, together with the nitrogen atom to which they are attached, a saturated (5- or 6-membered)heterocycle comprising, in addition to the nitrogen atom to which R₃ and R₄ are attached, 0 or 1 oxygen atom; said heterocycle being non-substituted or being substituted with a (=O) group.

More particularly, R₃ and R₄ can independently represent:
- a -(CH₂) group; or
- a -(C₂H₅) group;
or form, together with the nitrogen atom to which they are attached, a heterocycle selected from the group consisting of

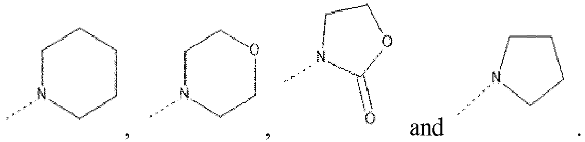

In a particular embodiment, the compound for its use according to the invention is selected from the following compounds: 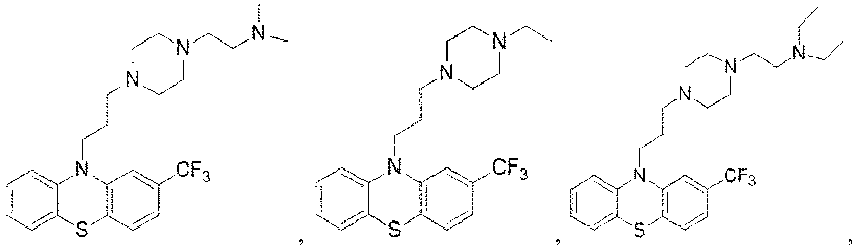 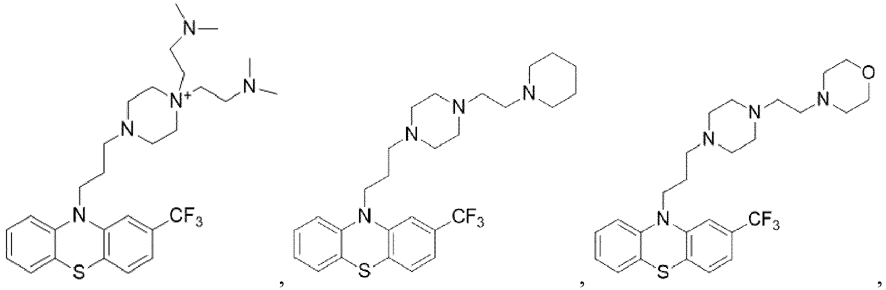 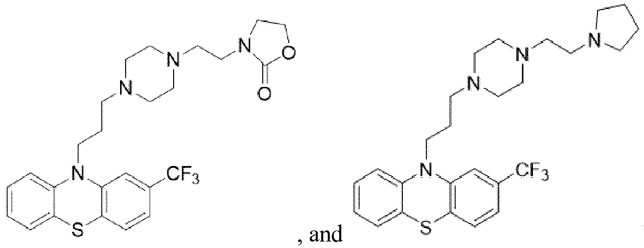 as well as their tautomeric and isomeric forms: racemic, enantiomeric and diastereoisomeric, and also their addition salts with inorganic and organic acids or with inorganic and organic bases.

In an embodiment of the invention, the tumor of interest is a pancreatic cancer.

Another object of the present invention is a pharmaceutical composition for its use in the treatment of a tumor selected from the group consisting of pancreatic cancer, liver cancer, melanoma, colon cancer, glioblastoma, osteosarcoma, prostate cancer and breast cancer,
wherein said composition comprises, in a pharmaceutically acceptable medium, at least one compound of formula (I).

In a particular embodiment, a pharmaceutical composition for its use according to the invention further comprises one or several anti-cancer drug(s) different from a compound as defined above.

Another object of the invention is a compound of formula (I) as defined here-above.

In a particular embodiment, said compound is selected from the following compounds: 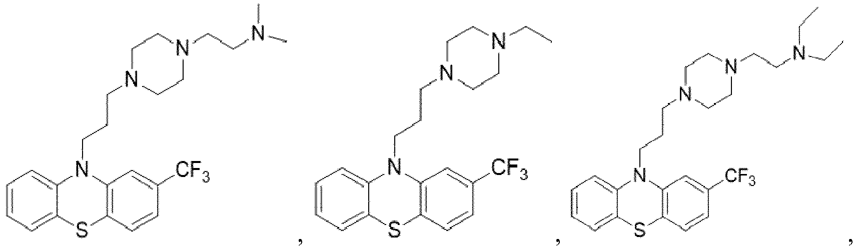 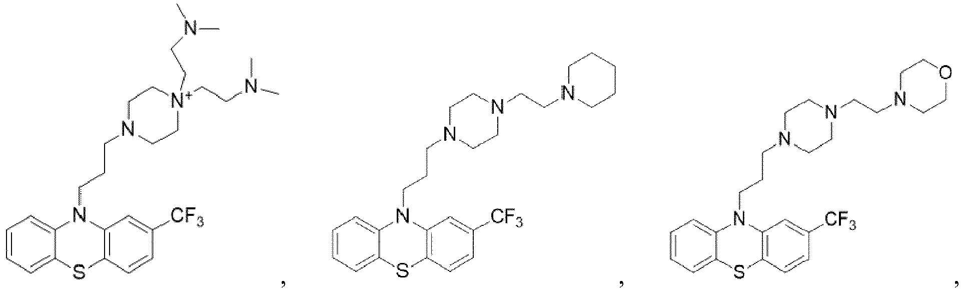 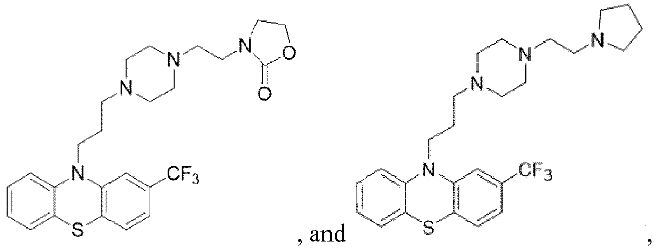 as well as their tautomeric and isomeric forms: racemic, enantiomeric and diastereoisomeric, and also their addition salts with inorganic and organic acids or with inorganic and organic bases.

More particularly, the compound of the invention can be selected from the group consisting of the following compounds: 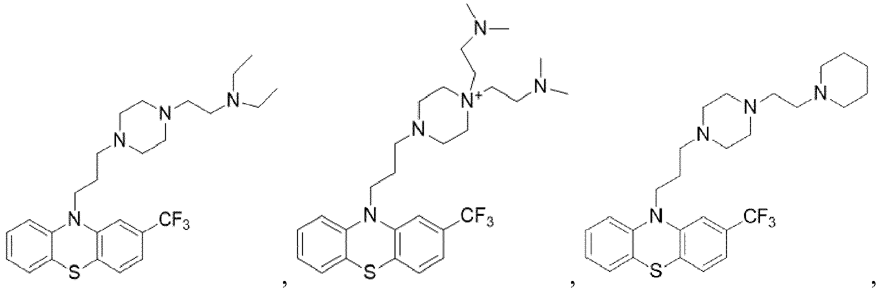 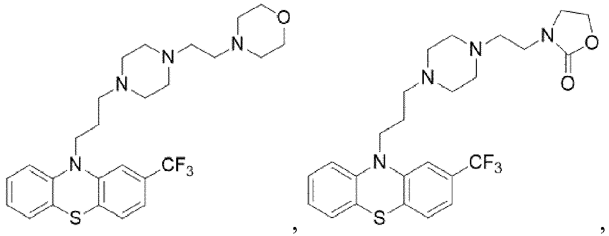 and 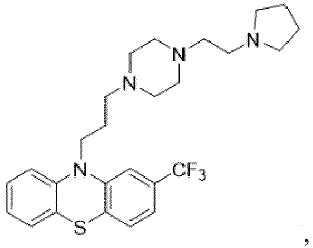 as well as their tautomeric and isomeric forms: racemic, enantiomeric and diastereoisomeric, and also their addition salts with inorganic and organic acids or with inorganic and organic bases.

### BRIEF DESCRIPTION OF THE FIGURES

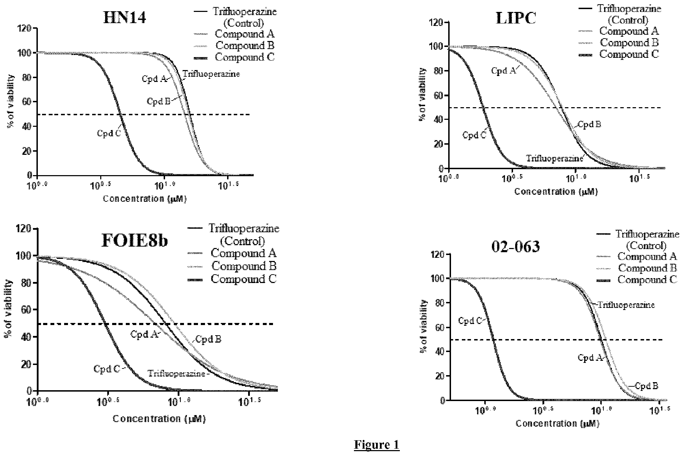

**Figure 1****:** Variation of the viability's percentage of four well characterized primary pancreatic cancer-derived cells 02.063 and LIPC (Basal subtype), Foie8b (derived from a liver metastasis) and HN14 (Classical subtype) after treatment with different concentrations of a control compound (Trifluoperazine) and three compounds according to the invention (Compounds A, B and C).
   Ordinate: % of cell viability.
   Abscissa: Concentration of the tested compound (µM).

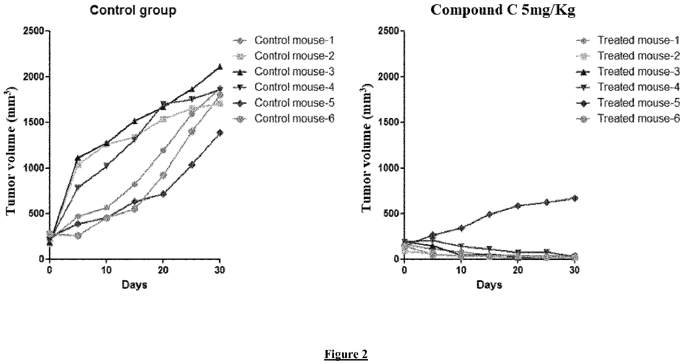

**Figure 2****:** Variation of the size the tumor of mice xenografted with 02.063 cells in the absence of any treatment (control - administration of a volume of vehicle solution equivalent to the volume of treatment administered to the treated mice) or treated with compound C of the invention (daily treatment of 30 days with 5 mg/kg of this compound). Day 0 on this figure corresponds to the day when the tumors reached 200 mm³ (so around 1 week after the injection of 15x10⁶ cells).
   Ordinate: Tumor volume (in mm³).
   Abscissa: number of days.

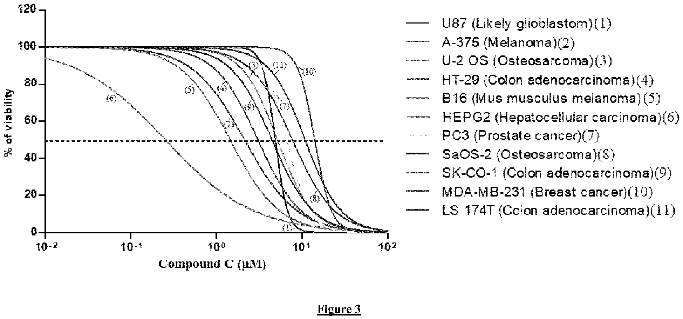

**Figure 3****:** variation of the viability's percentage of eleven cell lines derived from different tumors with increasing concentrations of Compound C: HEPG2 (Hepatocellular carcinoma), B16 (Mus musculus melanoma), A-375 (Melanoma), HT-29 (Colon adenocarcinoma), SK-CO-1 (Colon adenocarcinoma), U87 (Likely glioblastoma), U-2 OS (Osteosarcoma), PC3 (Prostate cancer), SaOS-2 (Osteosarcoma), LS 174T (Colon adenocarcinoma) and MDA-MB-231 (Breast cancer).
   Ordinate: % of cell viability.
   Abscissa: Concentration of Compound C (µM).

### DETAILED DESCRIPTION OF THE INVENTION

NUPR1, also known as p8 or Com1, is a stress-inducible 82-amino-acid-long, intrinsically disordered member of the AT-hook family of chromatin proteins.

NUPR1 binds to DNA in a similar manner to other chromatin proteins (Encinar et al., 2001, The Journal of biological chemistry 276, 2742-2751; Grasso et al., 2014, Cell death and differentiation 21, 1633-1641) so as to control the expression of gene targets (Hamidi et al., 2012, The Journal of clinical investigation 122, 2092-2103). At the cellular level, NUPR1 participates in many cancer-associated processes including cell-cycle regulation, apoptosis (Malicet et al., 2006a, Cell cycle 5, 829-830; Malicet et al., 2006b, Proceedings of the National Academy of Sciences of the United States of America 103, 2671-2676), cell migration and invasion (Sandi et al., 2011, Journal of cellular physiology 226, 3442-3451), development of metastases (Ree et al., 2000, Clinical cancer research : an official journal of the American Association for Cancer Research 6, 1778-1783) and DNA repair responses (Gironella et al., 2009, Journal of cellular physiology 221, 594-602). Indeed, NUPR1 has recently elicited significant attention due to its role in promoting cancer development and progression in the pancreas (Cano et al., 2014, Gut 63, 984-995; Hamidi et al., 2012, The Journal of clinical investigation 122, 2092-2103). Notably, NUPR1-dependent effects also mediate resistance to anticancer drugs (Giroux et al., 2006, Clinical cancer research: an official journal of the American Association for Cancer Research 12, 235-241; Palam et al., 2015, Cell death & disease 6, e1913; Tang et al., 2011, Oncology reports 25, 963-970).

It has moreover previously been showed that genetic inactivation of *Nupr1* antagonizes the growth of pancreatic cancer (Sandi et al., 2011, Journal of cellular physiology 226, 3442-3451; Vasseur et al., 2002, EMBO reports 3, 165-170), and that genetic inactivation of *Nupr1* stops the growth of hepatocarcinoma (Emma et al., 2016, Cell death & disease 7, e2269), non-small lung cancer (Guo et al., 2012, Anat Rec (Hoboken) 295, 2114-2121), cholangiocarcinoma (Kim *et al.,* 2012), glioblastoma (Li et al., 2017, J Neurooncol 132, 15-26), and multiple myeloma (Zeng et al., 2017, Chinese journal of cellular and molecular immunology 33, 1240-1246), thereby supporting a role for this protein as a promising therapeutic target for the development of therapies for cancer.

The mechanism by which the anticancer role of NUPR1 is played when its expression is knocked-down remains however still unclear.

Following a bottom-up approach, trifluoperazine, a well-known antipsychotic agent, has been recently identified as a ligand inducing a significantly different temperature denaturation profile in NUPR1 compared to control sample (NUPR1 with no compound added) (Neira et al., 2017, Scientific reports 7, 39732). The dissociation constant between NUPR1 and Trifluoperazine has in particular been found to be in the low micromolar range.

It has moreover been demonstrated that Trifluoperazine inactivates NUPR1, leading to cell senescence.

Accordingly, Trifluoperazine seemed an interesting compound as anti-cancer agent. However, as indicated in the examples of the present application, increasingly high doses of Trifluoperazine tested *in vivo* led to neurological effects on immunodeficient mice implanted with human pancreatic cancer cells-derived xenografts.

Thus, although Trifluoperazine seems relatively efficient as an anticancer agent, the neurological effect found in mice leaves it unusable in clinics.

This is why there remains a need for alternative compounds presenting an anticancer activity at least as good as the one of Trifluoperazine, but with less or preferably no side effects, and in particular with less or preferably no neurological side effects.

Surprisingly, the inventors have determined a group of compounds having anti-cancer properties at least equivalent to that of Trifluoperazine, but without any neurological side effect. Some of the compounds tested are even more effective than Trifluoperazine.

Considering the *in vitro* and *in vivo* experimental evidence provided in the present text, the inventors consider that tumors, and in particular cancers selected from the group consisting of pancreatic cancer, liver cancer, melanoma, colon cancer, glioblastoma, osteosarcoma, prostate cancer and breast cancer, and more particularly pancreatic cancer, can be treated through the use of at least a compound of formula (I) of the invention. Advantageously, said compound can be administered alone or in combination with other anti-cancer active agents.

In the sense of the invention:
- an alkyl group is a linear or branched saturated hydrocarbon-based aliphatic group comprising from 1 to 6 carbon atoms (and can be represented as a linear or branched (C₁-C₆)alkyl group). A linear or branched (C₁-C₆)alkyl group is thus a linear or branched saturated hydrocarbon-based aliphatic group comprising from 1 to 6 carbon atoms.

Examples that may be mentioned include methyl, ethyl, propyl, isopropyl, butyl, isobutyl, pentyl groups, and hexyl groups.
- a non-bonding pair is a pair of valence electrons that are not shared with another atom and can also be named a lone pair.
- a halogen atom can be selected from the group consisting of a fluorine, a chlorine, a bromine and an iodine atom.
- an alkoxy group is a radical -O-alkyl in which the alkyl group is as defined previously.
- an individual is preferably a mammal, and is in particular a human being. More particularly, an individual according to the invention has a cancer, and in particular a cancer selected from the group consisting of pancreatic cancer, liver cancer, melanoma, colon cancer, glioblastoma, osteosarcoma, prostate cancer and breast cancer, more particularly pancreatic cancer.

### Compounds for use according to the invention

The compounds of the invention are of formula (I): 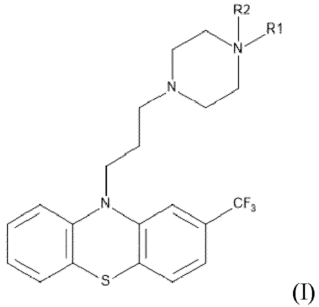 wherein:
R₁ represents a linear or branched (C₂-C₅)alkyl group, optionally substituted with a -NR₃R₄ group;
R₂ represents a non-bonding pair or a -(CH₂)₁₋₅NR₃R₄ group, wherein when R₂ does not represent a non-bonding pair, then the nitrogen atom to which R₂ is attached is positively charged; and
R₃ and R₄ independently represent:
   - a hydrogen atom;
   - a -OH group;
   - a linear or branched (C₁-C₆)alkyl group, said alkyl group being optionally substituted with one or several substituents chosen from a halogen atom, a -OH group and a -SH group;
   - a linear or branched (C₁-C₆)alkoxy group, said alkoxy group being optionally substituted with one or several substituents chosen from a halogen atom, a -OH group and a -SH group; or
   - a -COOH group;
or R₃ and R₄ form, together with the nitrogen atom to which they are attached, a saturated or unsaturated (3- to 6-membered)heterocycle comprising, in addition to the nitrogen atom to which R₃ and R₄ are attached, 0, 1 or 2 additional heteroatom(s) chosen from a nitrogen atom and an oxygen atom; said heterocycle being optionally substituted with one or several substituents chosen from a halogen atom, a -OH group, a (=O) group and a linear or branched (C₁-C₆)alkyl group;
said compound of formula (I) being in all the possible tautomeric and isomeric forms: racemic, enantiomeric and diastereoisomeric, and also as addition salts with inorganic and organic acids or with inorganic and organic bases of the compound of formula (I);
for its use in the treatment of a tumor selected from the group consisting of pancreatic cancer, liver cancer, melanoma, colon cancer, glioblastoma, osteosarcoma, prostate cancer and breast cancer.

In an embodiment, R₁ represents a linear or branched (C₂-C₅)alkyl group, preferably a linear (C₂-C₅)alkyl group, in particular a linear (C₂-C₄)alkyl group and more particularly a -C₂H₅ group.

In another embodiment, R₁ represents a linear or branched (C₂-C₅)alkyl group substituted with one -NR₃R₄ group, preferably a linear (C₂-C₅)alkyl group substituted with one -NR₃R₄ group, in particular a linear (C₂-C₄)alkyl group substituted with one -NR₃R₄ group and preferably represents a -(C₂H₄)NR₃R₄ group.

In a particular embodiment, R₁ represents:
- a -C₂H₅ group; or
- a -(C₂H₄)NR₃R₄ group, with R₃ and R₄ being as defined previously or as further defined here-after.

In a particular embodiment, a compound of the invention is of formula (II): 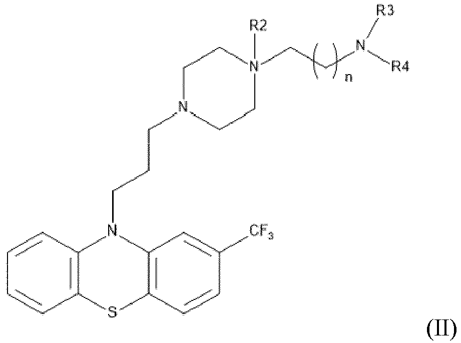 wherein:
n is an integer selected from the group ranging from 0 to 4; and
R₂, R₃ and R₄ are as defined previously or as defined here-after.
In an embodiment of the invention, n is 1.

In an embodiment, R₂ according to formulae (I) or (II) represents a non-bonding pair.

In another embodiment, R₂ according to formulae (I) or (II) represents a -(CH₂)₁₋₅NR₃R₄ group, in particular a -(CH₂)₂NR₃R₄ group, R₃ and R₄ being as defined above.

In a particular embodiment, R₂ according to formulae (I) or (II) represents a -(CH₂)₂NR₃R₄ group wherein R₃ and R₄ independently represent a linear or branched (C₁-C₆)alkyl group, preferably a linear (C₁-C₆)alkyl group and in particular a linear (C₁-C₃)alkyl group.

In a particular embodiment, R₂ according to formulae (I) or (II) represents a -(CH₂)₂NR₃R₄ group wherein R₃ and R₄ both represent a -CH₃ group.

In an embodiment of the invention, R₂ represents a non-bonding pair or a -(CH₂)₂N(CH₃)₂ group.

In every embodiment of the invention where R₂ is different from a non-bonding pair, the nitrogen atom to which R₂ is attached is positively charged.

In a particular embodiment, R₃ and R₄ are identical.

In an embodiment of the invention, R₃ and R₄ of formulae (I) or (II) independently represent a linear or branched (C₁-C₆)alkyl group or R₃ and R₄ form, together with the nitrogen atom to which they are attached, a saturated or unsaturated (3- to 6-membered)heterocycle comprising, in addition to the nitrogen atom to which R₃ and R₄ are attached, 0, 1 or 2 additional heteroatom(s) chosen from a nitrogen atom and an oxygen atom; said heterocycle being optionally substituted with one or several substituents chosen from a halogen atom, a -OH group, a (=O) group and a linear and branched (C₁-C₆)alkyl group.

In an embodiment of the invention, R₃ and R₄ independently represent a linear or branched (C₁-C₆)alkyl group, preferably a linear (C₁-C₆)alkyl group and in particular a linear (C₁-C₃)alkyl group. More particularly, R₃ and R₄ can both represent a -CH₃ group or a -C₂H₅ group, and they preferably both represent a -CH₃ group.

In another embodiment, R₃ and R₄ form, together with the nitrogen atom to which they are attached, a saturated or unsaturated (3- to 6-membered)heterocycle comprising, in addition to the nitrogen atom to which R₃ and R₄ are attached, 0, 1 or 2 additional heteroatom(s) chosen from a nitrogen atom and an oxygen atom; said heterocycle being optionally substituted with one or several substituents chosen from a halogen atom, a -OH group, a (=O) group and a linear and branched (C₁-C₆)alkyl group.

In a particular embodiment, said (3- to 6-membered)heterocycle is saturated.

In a particular embodiment, said heterocycle is a 5- or 6-membered heterocycle.

In an embodiment of the invention, said heterocycle comprises, in addition to the nitrogen atom to which R₃ and R₄ are attached, 0 or 1 additional heteroatom chosen from a nitrogen atom and an oxygen atom. If present, said additional heteroatom can in particular be an oxygen atom.

In particular, R₃ and R₄ can form, together with the nitrogen atom to which they are attached, a heterocycle selected from the group consisting of:

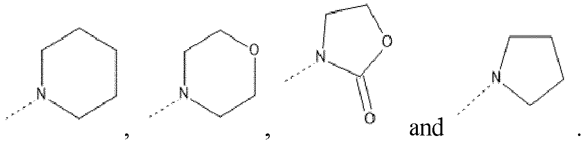

In a particular embodiment, said heterocycle is not substituted or is substituted with a (=O) group.

In an embodiment of the invention, R₃ and R₄ independently represent:
- a -(CH₂) group; or
- a -(C₂H₅) group;
or form, together with the nitrogen atom to which they are attached, a saturated (5- or 6-membered)heterocycle comprising, in addition to the nitrogen atom to which R₃ and R₄ are attached, 0 or 1 oxygen atom; said heterocycle being non-substituted or being substituted with a (=O) group.

In a particular embodiment, R₃ and R₄ independently represent:
- a -(CH₂) group; or
- a -(C₂H₅) group;
or form, together with the nitrogen atom to which they are attached, a heterocycle selected from the group consisting of:

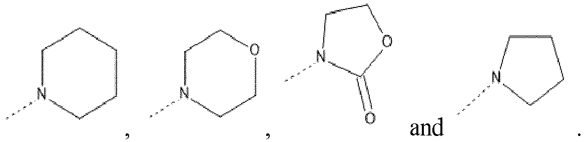

The compounds of the invention can in particular be selected among the following compounds:

| | |
|---|---|
| Compound A | 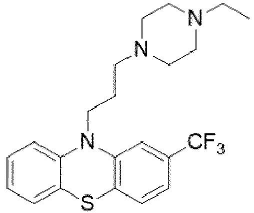 |
| Compound B (salt form of Compound A) | 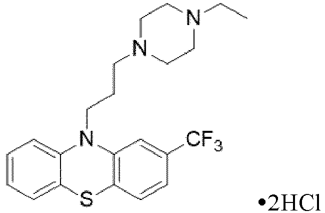 |
| Compound C | 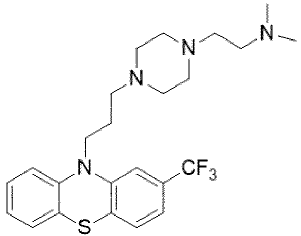 |
| Compound D | 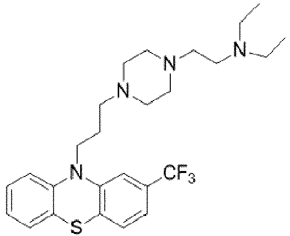 |
| Compound E | 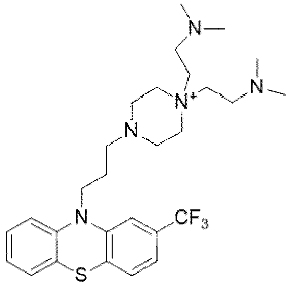 |
| Compound F | 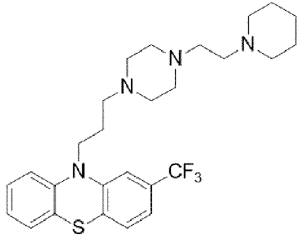 |
| Compound G | 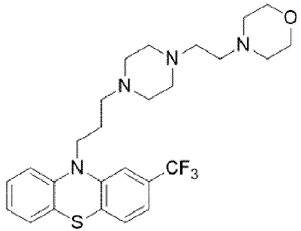 |
| Compound H | 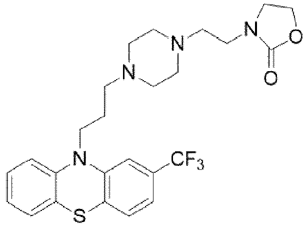 |
| Compound I | 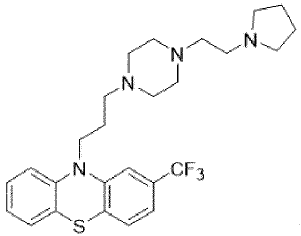 |

as well as their tautomeric and isomeric forms: racemic, enantiomeric and diastereoisomeric, and also their addition salts with inorganic and organic acids or with inorganic and organic bases.

In a particular embodiment, a compound for use according to the invention is preferably compound C:

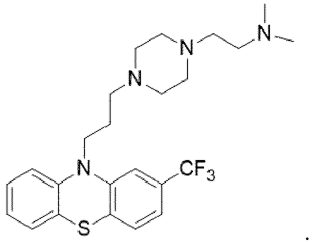

### Composition for use according to the invention

The present invention also relates to a pharmaceutical composition for its use in the treatment of a tumor selected from the group consisting of pancreatic cancer, liver cancer, melanoma, colon cancer, glioblastoma, osteosarcoma, prostate cancer and breast cancer, in an individual, said composition comprising, in a pharmaceutically acceptable medium, one or several compound of formula (I) as defined above as active agent(s).

Such composition comprises an effective dose of at least one compound according to the invention, or a pharmaceutically acceptable salt, and also at least one pharmaceutically acceptable medium or excipient.

A wide range of permissible dosages can be used and adjusted by the man skilled in the art depending of, for example, the nature of the cancer targeted, the weight, age and condition of the individual to be treated, etc. Doses administered to an individual can for example fall in the range from 1 µg/kg to 1 g/kg. The dosages may be single or divided, and may be administered according to a wide variety of protocols, including once a day, twice a day, three times a day, or even every other day, once a week or once a month. In each of these cases it is understood that the therapeutically effective amounts described herein correspond to the instance of administration, or alternatively to the total daily, weekly, month, or quarterly dose, as determined by the dosing protocol, of a compound according to formula (I) as active agent.

A pharmaceutically acceptable medium or excipient can be chosen, according to the pharmaceutical form and the mode of administration desired, from the usual excipients that are known to those skilled in the art.

A pharmaceutically acceptable medium or excipient is composed of materials that are considered safe and may be administered to an individual without causing undesirable biological side effects or unwanted interactions. Pharmaceutically acceptable mediums and their formulations are known to one skilled in the art and described, for example, in Remington's Pharmaceutical Sciences, (20th edition), ed. A. Gennaro, 2003, Lippincott Williams & Wilkins.

A composition of the invention can be administered to a subject with or without the aid of a delivery vehicle. Appropriate delivery vehicles for the compounds are known in the art and can be selected to suit the particular active agent.

For example, in some embodiments, the compounds of the invention can be incorporated into or encapsulated by, or bound to, a nanoparticle, microparticle, micelle, synthetic lipoprotein particle, or carbon nanotube.

The appropriate unit administration forms include oral-route forms such as tablets, soft or hard gel capsules, powders, granules and oral solutions or suspensions, sublingual, buccal, intratracheal, intraocular and intranasal administration forms, inhalation forms, transdermal, subcutaneous, intramuscular or intravenous administration forms, rectal administration forms and implants.

A composition according to the invention is preferably administered to an individual orally or parenterally.

In a pharmaceutical composition according to the invention, the compounds of formula (I) can be identical or can be a mixture of different compounds of formula (I).

In an embodiment of the invention, the composition further comprises one or several anti-cancer drug(s) different from a compound of formula (I).

Said anti-cancer drug can be selected from any anti-cancer drug known to the man skilled in the art.

Illustratively, said additional anti-cancer drug can be an antibody or antigen binding fragment thereof specific for a growth factor receptors or tumor specific antigens. Such growth factors receptors include, but are not limited to, epidermal growth factor receptor (EGFR; HER1); c-erbB2 (HER2); c-erbB3 (HER3); c-erbB4 (HER4); insulin receptor; insulin-like growth factor receptor 1 (IGF-1R); insulin-like growth factor receptor 2/Mannose-6-phosphate receptor (IGF-II R/M-6-P receptor); insulin receptor related kinase (IRRK); platelet-derived growth factor receptor (PDGFR); colony-stimulating factor-1 receptor (CSF-1R) (c-Fms); steel receptor (c-Kit); Flk2/Flt3; fibroblast growth factor receptor 1 (Flg/Cekl); fibroblast growth factor receptor 2 (Bek/Cek3/K-Sam); Fibroblast growth factor receptor 3; Fibroblast growth factor eceptor 4; nerve growth factor receptor (NGFR) (TrkA); BDNF receptor (TrkB); NT-3-receptor (TrkC); vascular endothelial growth factor receptor 1 (Flt1); vascular endothelial growth factor receptor 2/Flk1/KDR; hepatocyte growth factor receptor (HGF-R/Met); Eph; Eck; Eek; Cek4/Mek4/HEK; Cek5; Elk/Cek6; Cek7; Sek/Cek8; Cek9; Cek10; HEK11; 9 Ror1; Ror2; Ret; Axl; RYK; DDR; and Tie.

As additional anti-cancer drugs can also be mentioned conventional cancer therapeutics such as chemotherapeutic agents, immunotherapeutic agents, chemokines, cryotherapy, hormone therapy, and radiation therapy.

Representative chemotherapeutic agents include, but are not limited to, cisplatin, carboplatin, oxaliplatin, mechlorethamine, cyclophosphamide, chlorambucil, vincristine, vinblastine, vinorelbine, vindesine, taxol and derivatives thereof, irinotecan, topotecan, amsacrine, etoposide, etoposide phosphate, teniposide, epipodophyllotoxins, trastuzumab (HERCEPTIN®), cetuximab, and rituximab (RITUXAN® or MABTHERA®), bevacizumab (AVASTIN®), and combinations thereof.

In some embodiments, a composition according to the invention further comprises an immunotherapeutic agent, different from a compound of formula (I) according to the invention.

The term "immunotherapeutic agent," as used herein, refers to a compound, composition or treatment that indirectly or directly enhances, stimulates or increases the body's immune response against cancer cells and/or that decreases the side effects of other anticancer therapies. Immunotherapy is thus a therapy that directly or indirectly stimulates or enhances the immune system's responses to cancer cells and/or lessens the side effects that may have been caused by other anti-cancer agents. Immunotherapy is also referred to in the art as immunologic therapy, biological therapy biological response modifier therapy and biotherapy.

Examples of common immunotherapeutic agents known in the art include, but are not limited to, cytokines, cancer vaccines, monoclonal antibodies and non-cytokine adjuvants. Alternatively the immunotherapeutic treatment may consist of administering the subject with an amount of immune cells (T cells, NK, cells, dendritic cells, B cells...).

Immunotherapeutic agents can be non-specific, i.e. boost the immune system generally so that the human body becomes more effective in fighting the growth and/or spread of cancer cells, or they can be specific, i.e. targeted to the cancer cells themselves immunotherapy regimens may combine the use of non-specific and specific immunotherapeutic agents. Non-specific immunotherapeutic agents are substances that stimulate or indirectly improve the immune system. Non-specific immunotherapeutic agents have been used alone as a main therapy for the treatment of cancer, as well as in addition to a main therapy, in which case the non-specific immunotherapeutic agent functions as an adjuvant to enhance the effectiveness of other therapies (e.g. cancer vaccines). Non-specific immunotherapeutic agents can also function in this latter context to reduce the side effects of other therapies, for example, bone marrow suppression induced by certain chemotherapeutic agents. Non-specific immunotherapeutic agents can act on key immune system cells and cause secondary responses, such as increased production of cytokines and immunoglobulins. Alternatively, the agents can themselves comprise cytokines. Non-specific immunotherapeutic agents are generally classified as cytokines or non-cytokine adjuvants.

A number of cytokines have found application in the treatment of cancer either as general non-specific immunotherapies designed to boost the immune system, or as adjuvants provided with other therapies. Suitable cytokines include, but are not limited to, interferons, interleukins and colony-stimulating factors. Interferons (IFNs) contemplated by the present invention include the common types of IFNs, IFN-alpha (IFN-α), IFN-beta (IFN-β) and IFN-gamma (IFN-γ). IFNs can act directly on cancer cells, for example, by slowing their growth, promoting their development into cells with more normal behaviour and/or increasing their production of antigens thus making the cancer cells easier for the immune system to recognise and destroy.

IFNs can also act indirectly on cancer cells, for example, by slowing down angiogenesis, boosting the immune system and/or stimulating natural killer (NK) cells, T cells and macrophages. Recombinant IFN-alpha is available commercially as Roferon (Roche Pharmaceuticals) and Intron A (Schering Corporation).

Interleukins contemplated by the present invention include IL-2, IL-4, IL-11 and IL-12. Examples of commercially available recombinant interleukins include Proleukin® (IL-2; Chiron Corporation) and Neumega® (IL-12; Wyeth Pharmaceuticals). Zymogenetics, Inc. (Seattle, Wash.) is currently testing a recombinant form of IL-21, which is also contemplated for use in the combinations of the present invention. Colony-stimulating factors (CSFs) contemplated by the present invention include granulocyte colony stimulating factor (G-CSF or filgrastim), granulocyte-macrophage colony stimulating factor (GM-CSF or sargramostim) and erythropoietin (epoetin alfa, darbepoietin). Treatment with one or more growth factors can help to stimulate the generation of new blood cells in subjects undergoing traditional chemotherapy. Accordingly, treatment with CSFs can be helpful in decreasing the side effects associated with chemotherapy and can allow for higher doses of chemotherapeutic agents to be used. Various-recombinant colony stimulating factors are available commercially, for example, Neupogen® (G-CSF; Amgen), Neulasta (pelfilgrastim; Amgen), Leukine (GM-CSF; Berlex), Procrit (erythropoietin; Ortho Biotech), Epogen (erythropoietin; Amgen), Arnesp (erytropoietin).

In addition to having specific or non-specific targets, immunotherapeutic agents can be active, i.e. stimulate the body's own immune response, or they can be passive, i.e. comprise immune system components that were generated external to the body. Passive specific immunotherapy typically involves the use of one or more monoclonal antibodies that are specific for a particular antigen found on the surface of a cancer cell or that are specific for a particular cell growth factor. Monoclonal antibodies may be used in the treatment of cancer in a number of ways, for example, to enhance a subject's immune response to a specific type of cancer, to interfere with the growth of cancer cells by targeting specific cell growth factors, such as those involved in angiogenesis, or by enhancing the delivery of other anticancer agents to cancer cells when linked or conjugated to agents such as chemotherapeutic agents, radioactive particles or toxins.

Monoclonal antibodies currently used as cancer immunotherapeutic agents that are suitable for inclusion in the combinations of the present invention include, but are not limited to, rituximab (Rituxan®), trastuzumab (Herceptin®), ibritumomab tiuxetan (Zevalin®), tositumomab (Bexxar®), cetuximab (C-225, Erbitux®), bevacizumab (Avastin®), gemtuzumab ozogamicin (Mylotarg®), alemtuzumab (Campath®), and BL22. Other examples include anti-CTLA4 antibodies (e.g. Ipilimumab), anti-PDl antibodies, anti-PDLl antibodies, anti-TIMP3 antibodies, anti-LAG3 antibodies, anti-B7H3 antibodies, anti-B7H4 antibodies or anti-B7H6 antibodies.

In some embodiments, antibodies include B cell depleting antibodies. Typical B cell depleting antibodies include but are not limited to anti-CD20 monoclonal antibodies [e.g. Rituximab (Roche), Ibritumomab tiuxetan (Bayer Schering), Tositumomab (GlaxoSmithKline), AME-133v (Applied Molecular Evolution), Ocrelizumab (Roche), Ofatumumab (HuMax-CD20, Gemnab), TRU-015 (Trubion), and IMMU-106 (Immunomedics)], an anti-CD22 antibody [e.g. Epratuzumab, Leonard et al., Clinical Cancer Research (Z004) 10: 53Z7-5334], anti-CD79a antibodies, anti-CD27 antibodies, or anti-CD19 antibodies (e.g. U.S. Pat. No. 7,109,304), anti-BAFF-R antibodies (e.g. Belimumab, GlaxoSmithKline), anti-APRIL antibodies (e.g. anti-human APRIL antibody, ProSci inc.), and anti-IL-6 antibodies [e.g. previously described by De Benedetti et al., J Immunol (2001) 166: 4334-4340 and by Suzuki et al., Europ J of Immunol (1992) 22 (8) 1989-1993]. Can also be mentioned Nivolumab (Bristol-Myers Squibb), Pembrolizumab (Msd france) and Ipilimumab (Bristol myers squibb eeig).

The immunotherapeutic treatment may consist of allografting, in particular, allograft with hematopoietic stem cell HSC. The immunotherapeutic treatment may also consist in an adoptive immunotherapy as described by Nicholas P. Restifo, Mark E. Dudley and Steven A. Rosenberg "Adoptive immunotherapy for cancer: harnessing the T cell response, Nature Reviews Immunology, Volume 12, April 2012). In adoptive immunotherapy, the subject's circulating lymphocytes, NK cells, are isolated amplified in vitro and readministered to the subject. The activated lymphocytes or NK cells are most preferably the subject's own cells that were earlier isolated from a blood or tumor sample and activated (or "expanded") *in vitro.*

The present invention also relates to a method for treating a cancer, comprising the administration to an individual in need thereof, of at least one compound of formula (I) as described above, said cancer being in particular selected from the group consisting of pancreatic cancer, liver cancer, melanoma, colon cancer, glioblastoma, osteosarcoma, prostate cancer and breast cancer.

The present invention also relates to a method for treating a cancer, comprising the administration to an individual in need thereof, of at least a composition as described above, said cancer being in particular selected from the group consisting of pancreatic cancer, liver cancer, melanoma, colon cancer, glioblastoma, osteosarcoma, prostate cancer and breast cancer.

The present invention also relates to the use of at least one compound of formula (I) as described above, or of a composition as described above, for the treatment of a cancer in an individual, said cancer being in particular selected from the group consisting of pancreatic cancer, liver cancer, melanoma, colon cancer, glioblastoma, osteosarcoma, prostate cancer and breast cancer.

The present invention also relates to the use of at least one compound of formula (I) as described above, or of a composition as described above, for the preparation of a medicament for the treatment of a cancer in an individual, said cancer being in particular selected from the group consisting of pancreatic cancer, liver cancer, melanoma, colon cancer, glioblastoma, osteosarcoma, prostate cancer and breast cancer.

### EXAMPLES

### Synthesis of the compounds according to the invention

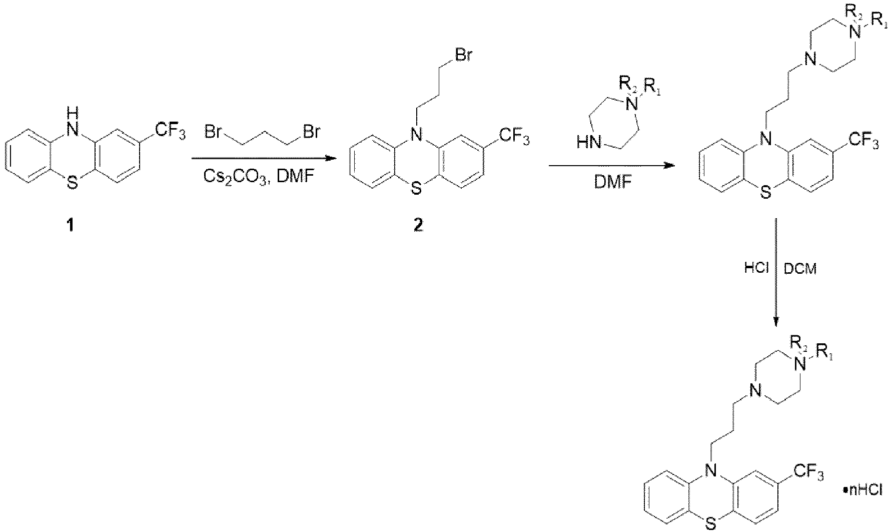

The 2-(trifluoromethyl)-10H-phenothiazine (compound 1 in the above schema, 204 mg), 1,3-dibromepropane (0.4 mL) and Cs₂CO₃ (150 mg) were dissolved in dimethylformamide (DMF) (1.6 mL) and stirred at 65°C for 12 h.

After completion of the reaction, the solvent was removed.

The residue was dissolved in ethyl acetate and washed with brine.

The combined organic layer was dried over Na₂SO₄, filtered and concentrated under reduced pressure to yield crude product which was further purified by column chromatography to afford compound 2 (96 mg, 31.8%). Then, compound 2 and piperazine (or other piperazine analogues) were dissolved in DMF and stirred at 25°C overnight.

After completion of the reaction, the solvent was removed and the residue was purified by column chromatography to afford the Trifluoperazine-derived compounds.

The yields of compounds A and C were for example respectively 77% and 64%.

In order to obtain the salts of the compounds of the invention, the compounds were then completely dissolved in CH₂Cl₂ at 25°C. The anhydrous HCl was bubbled through the solution for 5 minutes. Then the solvent was removed and the residue was purified by column chromatography to afford the hydrochloride salt of the compounds of the invention with the yield of 99%.

### Biophysical and biochemical characteristics of the compounds of the invention

A three-part approach was used:
(a) Fluorescence studies: A two-part strategy was followed:
   i/ acquisition of the intrinsic fluorescence spectra of NUPR1 (which only contains two Tyr residues) in the absence and in the presence of the compounds; and
   ii/ acquisition of the fluorescence spectra of the probe ANS (8-anilinonaphthalene-1-sulfonic acid) when NUPR1 was isolated in solution and in the presence of the compounds.

In both strategies, it was monitored whether there were changes in the environment around the two Tyr residues in NUPR1, or in the environment around the NUPR1 pocket where ANS is allocated in the presence of any of the drugs.

The results show that:
i/ the region around Tyr30 and Tyr36 of NUPR1 changed in the presence of any of the compounds; and
ii/ the ANS fluorescence also changed in the presence of the compounds.

Thus, it can be concluded that there was binding of NUPR1 to any of the compounds and the binding occurred around the 30s region of the sequence and altered the solvent-exposed hydrophobic patches of NUPR1. Experiments were carried out at 25°C and pH 7.4.
(b) NMR studies: Once it was spectroscopically determined that there was binding between NUPR1 and the compounds of the invention, we aimed to determine whether the NUPR1 binding region was the same for all of them. 2D ¹H- ¹⁵N- HSQC NMR experiments were carried out, with 15N-labelled NUPR1, and for all the compounds there were not changes in the chemical shifts of the cross-peaks, but rather variations in their intensities upon binding. All the compounds affected the signals of a similar set of residues: those around the 20s and those at the C terminal region of the protein. Experiments were carried out at pH 4.5 and 25°C to decrease solvent-exchange of amide protons.
(c) ITC studies: Target engagement for the different compounds was assessed by isothermal titration calorimetry (the gold standard technique in binding affinity determination), which allowed the thermodynamic characterization of the NUPR1-compound interaction.

The interaction between compounds of the invention and NUPR1 was studied by isothermal titration calorimetry, and the affinity, enthalpy and stoichiometry of binding were determined at 25°C.

Titrations were performed in an AutoiTC200 (MicroCal, Malvern Instruments). A solution of NUPR1 (20 µM, in sodium phosphate 20 mM pH 7.0, 1% DMSO) in the calorimetric cell was titrated with a solution of corresponding compound (200 µM, in sodium phosphate 20 mM pH 7.0, 1 % DMSO) located in the injecting syringe.

Because compounds were initially dissolved as concentrated (∼10 mM) stock solutions in pure DMSO, a residual percentage of DMSO was present in the final solutions. It has been demonstrated that, in general, a low percentage (1-3 %) of DMSO does not interfere in protein-ligand interactions. A standard protocol was employed: 19 titrant injections with 2 µL were programmed with a spacing of 150s, a stirring speed of 750 rpm, and a reference power of 10 µcal/s. From the thermogram (thermal power as a function of time), the binding isotherm (ligand-normalized heats as a function of the molar ratio) was obtained through integration of the individual heat effect associated with each ligand injection. Non-linear least squares regression analysis employing a model considering a single binding site in NUPR1 allowed the estimation of the binding parameters: dissociation constant, biding enthalpy, and stoichiometry. All compounds exhibited dissociation constants in the low micromolar range (K_{d} of compound A: 11 µM; K_{d} of compound B: 7.1 µM). However, compound C was the stronger binder, with a dissociation constant close to 2 µM (K_{d} of 2.1 µM).

### Biological characterization of compounds of the invention

The biological effect *in vitro* of the compounds A, B and C were then studied on four well characterized primary pancreatic cancer-derived cells 02.063 and LIPC (Basal subtype), Foie8b (derived from a liver metastasis) and HN14 (Classical subtype).

The cells were screened for chemosensitivity to these compounds for 72 h with increasing concentrations of the compounds ranging from 0 to 100 µM in 96-well plates. Trifluoperazine was used as the reference compound. Each experiment was performed in triplicate and repeated at least three times. Cell viability was estimated after addition of the PrestoBlue cell viability reagent (Life Technologies) for 3 h.

Treatment of 02.063, LIPC, Foie8b and HN14 cells with all these compounds showed that the cell death induced with compound A and compound B was close to that induced by Trifluoperazine; whereas treatment with compound C was around 10-times more efficient than with Trifluoperazine (see Figure 1).

Altogether, these data strongly suggest that:
i/ the effect of these compounds seems to be independent of the subtype of pancreatic cancer cells;
ii/ Compound C is the most efficient compound to kill the pancreatic cancer cells; and
iii/ compounds A and B have similar affinity to that of Trifluoperazine.

Because compound C was the most efficient *in vitro,* this compound was selected to treat mice xenografted with 02.063 cells.

02.063 cells were inoculated subcutaneously (15 x 10⁶ cells) in NMRI-Nude 8-week old mice. When tumors reached 200 mm³, (around 1 week after the injection), a daily treatment was started for 30 days with 5 mg/kg of the compound C and the control group receiving an equivalent volume of vehicle solution (6 mice in control group, 6 mice treated with compound C).

As expected, tumor volumes increased in an exponential manner in control mice. In contrast, when the mice were treated with compound C, the tumor stopped the growth almost immediately, and surprisingly, the size of the tumors decreased progressively to almost disappear after 20 days of treatment (see Figure 2).

A similar experiment was performed with Trifluoperazine (data not shown). With the lowest dose of Trifluoperazine, the tumor volume increased only 50% compared to the control during the same period, and at higher dose, the tumor growth was rapidly, and almost completely, stopped. However, increasingly high doses of Trifluoperazine led to neurological effect on treated mice such has lethargy. Although relatively efficient as an anticancer agent, the neurological effect found in mice left Trifluoperazine unusable in clinics.

On the contrary, the treatment with compound C did not show any appreciable neurological effect, including in a chronic treatment with 10 mg/kg.

### Compound C acts as antitumor agent in other tumors

The effect of compound C was also evaluated by treating cellular lines derived from different tumors with increasing concentrations of this compound. The protocol applied is the same as the one mentioned above for cell lines 02-063, HN14, LIPC and Foie8b.

The evaluation of cells such as U87 (glioblastoma), A375 and B16 (melanoma), U2OS and SaOS (osteosarcoma), HT29, SK-CO-1 and LS174T (colon cancer), HepG2 (hepatocarcinoma), PC3 (prostate) and MDA-MB-231 (breast cancer), demonstrated that compound C is efficient to kill the tumors cells with a range of IC₅₀ from 0.25 µM (HepG2 cells) to 14.12 µM (MDA-MB-231) as presented in Figure 3.

The IC₅₀ values obtained are represented in the following Table 2.

| **Tumor type** | **IC50 value (µM)** |
|---|---|
| **U87** | 4,877 |
| **A-375** | 2,137 |
| **U-2 OS** | 5,173 |
| **HT-29** | 3,063 |
| **B16** | 1,452 |
| **HEPG2** | 0,254 |
| **PC3** | 6,667 |
| **SaOS-2** | 7,745 |
| **SK-CO-1** | 4,466 |
| **MDA-MB-231** | 14,120 |
| **LS 174T** | 10,910 |

## Claims

1. A compound of formula (I): 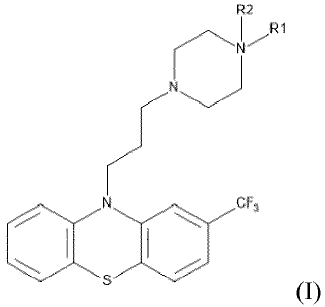 wherein:
R₁ represents a linear or branched (C₂-C₅)alkyl group, optionally substituted with a -NR₃R₄ group;
R₂ represents a non-bonding pair or a -(CH₂)₁₋₅NR₃R₄ group, wherein when R₂ does not represent a non-bonding pair, then the nitrogen atom to which R₂ is attached is positively charged; and
R₃ and R₄ independently represent:
- a hydrogen atom;
- a -OH group;
- a linear or branched (C₁-C₆)alkyl group, said alkyl group being optionally substituted with one or several substituents chosen from a halogen atom, a -OH group and a -SH group;
- a linear or branched (C₁-C₆)alkoxy group, said alkoxy group being optionally substituted with one or several substituents chosen from a halogen atom, a -OH group and a -SH group; or
- a -COOH group;
or R₃ and R₄ form, together with the nitrogen atom to which they are attached, a saturated or unsaturated (3- to 6-membered)heterocycle comprising, in addition to the nitrogen atom to which R₃ and R₄ are attached, 0, 1 or 2 additional heteroatom(s) chosen from a nitrogen atom and an oxygen atom; said heterocycle being optionally substituted with one or several substituents chosen from a halogen atom, a -OH group, a (=O) group and a linear or branched (C₁-C₆)alkyl group;
said compound of formula (I) being in all the possible tautomeric and isomeric forms: racemic, enantiomeric and diastereoisomeric, and also as addition salts with inorganic and organic acids or with inorganic and organic bases of the compound of formula (I); for its use in the treatment of a tumor selected from the group consisting of pancreatic cancer, liver cancer, melanoma, colon cancer, glioblastoma, osteosarcoma, prostate cancer and breast cancer.

2. The compound for its use according to claim 1, wherein R₁ represents:
- a -C₂H₅ group; or
- a -(C₂H₄)NR₃R₄ group, with R₃ and R₄ being as defined in claim 1.

3. The compound for its use according to claim 1 or 2, wherein said compound is of formula (II): 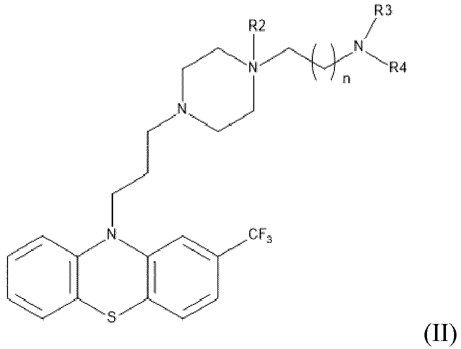 wherein:
n is an integer selected from the group ranging from 0 to 4; and
R₂, R₃ and R₄ are as defined in claim 1.

4. The compound for its use according to claim 3, wherein n is 1.

5. The compound for its use according to any one of claims 3 and 4, wherein R₃ and R₄ independently represent a linear or branched (C₁-C₆)alkyl group or R₃ and R₄ form, together with the nitrogen atom to which they are attached, a saturated or unsaturated (3- to 6-membered)heterocycle comprising, in addition to the nitrogen atom to which R₃ and R₄ are attached, 0, 1 or 2 additional heteroatom(s) chosen from a nitrogen atom and an oxygen atom;
said heterocycle being optionally substituted with one or several substituents chosen from a halogen atom, a -OH group, a (=O) group and a linear and branched (C₁-C₆)alkyl group.

6. The compound for its use according to any one of claims 3 to 5, wherein:
R₃ and R₄ independently represent:
- a -(CH₂) group; or
- a -(C₂H₅) group;
or form, together with the nitrogen atom to which they are attached, a saturated (5- or 6-membered)heterocycle comprising, in addition to the nitrogen atom to which R₃ and R₄ are attached, 0 or 1 oxygen atom; said heterocycle being non-substituted or being substituted with a (=O) group.

7. The compound for its use according to any one of claims 3 to 6, wherein:
R₃ and R₄ independently represent:
- a -(CH₂) group; or
- a -(C₂H₅) group;
or form, together with the nitrogen atom to which they are attached, a heterocycle selected from the group consisting of:

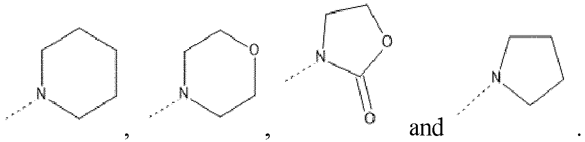

8. The compound for its use according to any one of claims 1 to 7, wherein said compound is selected from the following compounds: 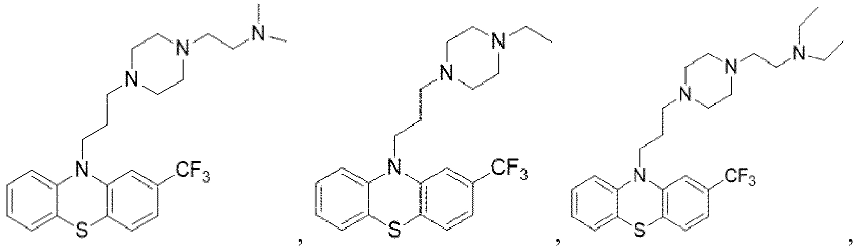 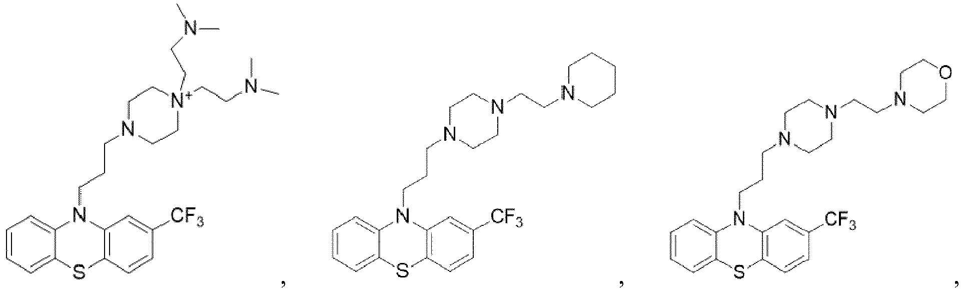 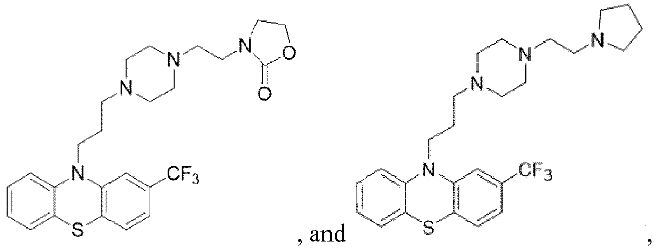 as well as their tautomeric and isomeric forms: racemic, enantiomeric and diastereoisomeric, and also their addition salts with inorganic and organic acids or with inorganic and organic bases.

9. The compound for its use according to any one of claims 1 to 8, wherein the tumor is a pancreatic cancer.

10. A pharmaceutical composition for its use in the treatment of a tumor selected from the group consisting of pancreatic cancer, liver cancer, melanoma, colon cancer, glioblastoma, osteosarcoma, prostate cancer and breast cancer, in an individual,
wherein said composition comprises, in a pharmaceutically acceptable medium, at least one compound as defined in any one of claims 1 to 9.

11. The pharmaceutical composition for its use according to claim 10, further comprising one or several anti-cancer drug(s) different from a compound as defined in anyone of claims 1 to 9.

12. A compound of formula (I): 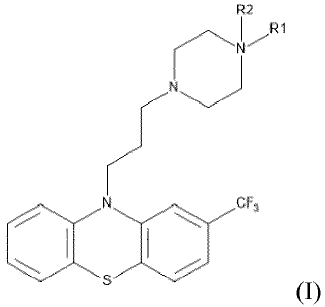 wherein:
R₁ represents a linear or branched (C₂-C₅)alkyl group, optionally substituted with a -NR₃R₄ group;
R₂ represents a non-bonding pair or a -(CH₂)₁₋₅NR₃R₄ group, wherein when R₂ does not represent a non-bonding pair, then the nitrogen atom to which R₂ is attached is positively charged; and
R₃ and R₄ independently represent:
- a hydrogen atom;
- a -OH group;
- a linear or branched (C₁-C₆)alkyl group, said alkyl group being optionally substituted with one or several substituents chosen from a halogen atom, a -OH group and a -SH group;
- a linear or branched (C₁-C₆)alkoxy group, said alkoxy group being optionally substituted with one or several substituents chosen from a halogen atom, a -OH group and a -SH group; or
- a -COOH group;
or R₃ and R₄ form, together with the nitrogen atom to which they are attached, a saturated or unsaturated (3- to 6-membered)heterocycle comprising, in addition to the nitrogen atom to which R₃ and R₄ are attached, 0, 1 or 2 additional heteroatom(s) chosen from a nitrogen atom and an oxygen atom; said heterocycle being optionally substituted with one or several substituents chosen from a halogen atom, a -OH group, a (=O) group and a linear or branched (C₁-C₆)alkyl group;
said compound of formula (I) being in all the possible tautomeric and isomeric forms: racemic, enantiomeric and diastereoisomeric, and also as addition salts with inorganic and organic acids or with inorganic and organic bases of the compound of formula (I).

13. Compound according to claim 12, selected from the following compounds: 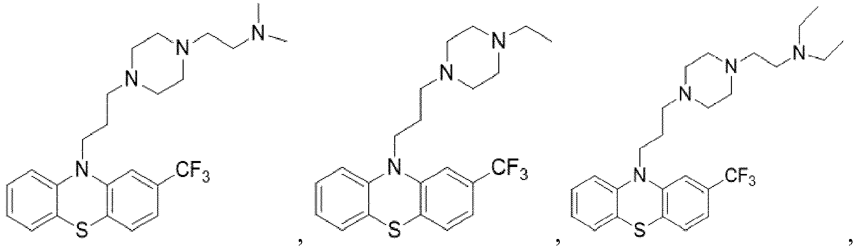 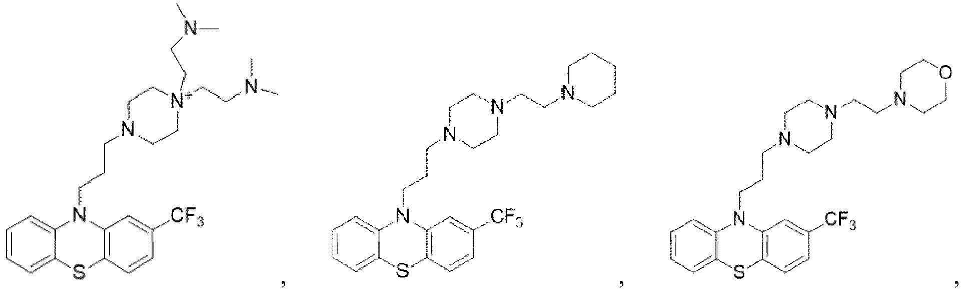 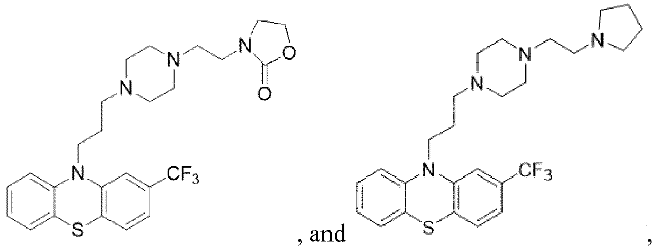 as well as their tautomeric and isomeric forms: racemic, enantiomeric and diastereoisomeric, and also their addition salts with inorganic and organic acids or with inorganic and organic bases.

14. Compound according to claim 12 or 13, selected from the group consisting of the following compounds: 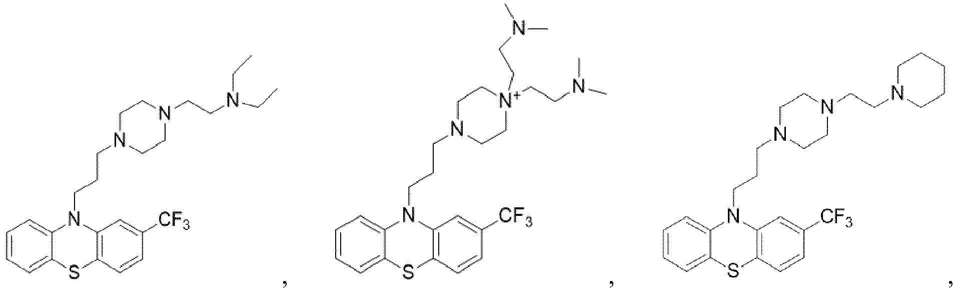 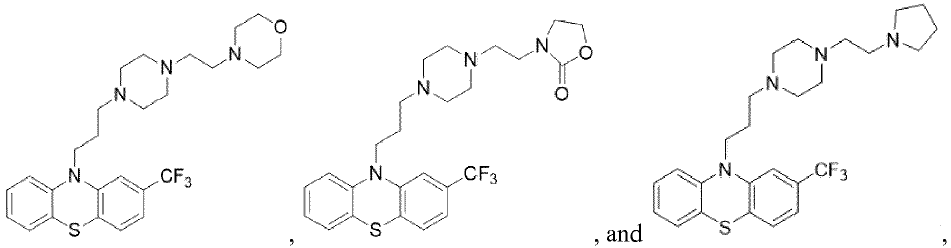 as well as their tautomeric and isomeric forms: racemic, enantiomeric and diastereoisomeric, and also their addition salts with inorganic and organic acids or with inorganic and organic bases.
